# EUROPEAN PATENT APPLICATION

(11) **EP 4 545 959 A1**
(43) Date of publication of application: **30.04.2025**
(21) Application number: 23831853.9
(22) Date of filing: 27.06.2023
(51) Int. Cl.: G01N 27/327, G01N 33/483

(54) **BIOSENSOR STRIP FOR MEASURING BIO SAMPLE, ELECTRODE STRUCTURE THEREOF, AND BIO SAMPLE MEASUREMENT METHOD USING SAME**

(30) Priority: 27.06.2022 KR 20220077952
(71) Applicant: Dong Woon Anatech Co., Ltd, Seoul 06716 (KR)
(72) Inventor: KWON, Min Su, Seoul 06716 (KR); KYE, Ji Won, Seoul 06716 (KR); IM, Eun Hye, Seoul 06716 (KR); KIM, Mi Rim, Seoul 06716 (KR); JANG, In Su, Seoul 06716 (KR); CHO, Hyun Seok, Seoul 06716 (KR); KIM, Dong Cheol, Seoul 06716 (KR)
(74) Representative: HGF
(86) International application number: PCT/KR2023/008900
(87) International publication number: WO 2024/005493

(57) **Abstract**

Disclosed are a biosensor strip for measuring a specimen, an electrode structure thereof, and a specimen measurement method using the same. An electrode structure of a biosensor strip according to one embodiment may comprise: a strip recognition electrode used to recognize that the biosensor strip is inserted into a specimen measurement device; a specimen recognition electrode used to recognize a specimen; and a working electrode and a working reference electrode used to measure the specimen.

## Description

### [TECHNICAL FIELD]

Embodiments of the present disclosure described herein relate to a biosensor strip for specimen measurement, an electrode structure thereof, and specimen measurement method using the same.

### [BACKGROUND ART]

Quantitatively or qualitatively analyzing the analytes present in a biological sample is chemically and clinically important. For example, representative examples include measuring blood glucose levels in blood for diabetic patients or measuring cholesterol, which is a factor for various adult diseases.

As known in the art, in electrochemical biosensors using enzyme activity, it is very important to more quickly and reproducibly measure enzyme activity of specific substances in the biological samples (hereinafter, referred to as "specimens"), such as saliva or blood, glucose, uric acid, protein, DNA(DeoxyriboNucleic Acid), and sucrose in clinical chemistry tests, and GOT(Glutamate-Oxaloacetate Transaminase) or GPT(Glutamate-Pyruvate Transaminase) in liver function tests. Here, the biosensor is composed of an identification part that identifies a measurement subject and a conversion part that performs conversion into an electrical signal.

A biological material is used in the identification part, and when the biological material recognizes the measurement subject, chemical changes or physical changes occur. A part that converts these changes into electrical signals is the conversion part, and the identification part and the conversion part are collectively called biosensor electrodes.

A currently commercialized measurement method for a strip-type biosensor is commonly a method of bringing the specimen into contact with a specimen insertion passage of the biosensor, introducing the specimen into the specimen insertion passage, and accumulating the specimen in the specimen insertion passage by using capillary phenomenon that is made possible through plasma or chemical surfactant treatment process and is a stronger force than the earth's gravity during a manufacturing process, and then applying a specimen measurement signal to a working electrode and a working reference electrode to measure a response signal for the specimen.

In conventional biosensors, to recognize whether the specimen is in contact with the biosensor strip prior to measuring the specimen, time points of specimen contact and introduction may be recognized by applying a specimen recognition signal to the working electrode and the working reference electrode.

However, in this case, since the working electrode and the working reference electrode that should be used in a process of measuring the specimen are also used in a process of recognizing whether the specimen is in contact with the biosensor strip, electric double layers (EDL) may be formed on surfaces of the working electrode and the working reference electrode even before measuring the specimen. The electric double layer appears when an electric field is applied to a boundary between different materials (electrodes, specimens, or solutions), and electric double layer capacitance (DLC) that is a capacitance of the electric double layer may be included in a response current signal for the specimen to be measured by applying the specimen measurement signal, even if it is a very small amount. Thus, the existing technique that forms the electric double layers on the working electrode and the working reference electrode before measuring the specimen has a problem of degraded specimen measurement accuracy.

Accordingly, there is a need to propose a technology for solving problems that the conventional biosensor have.

### [DETAILED DESCRIPTION OF THE INVENTION]

### [TECHINICAL PROBLEM]

Some embodiments provide an electrode structure of a biosensor strip including a strip recognition electrode and a specimen recognition electrode that are separately provided to operate electrically independent of a working electrode and a working reference electrode to improve specimen measurement accuracy by preventing an electric double layer from being formed on surfaces of the working electrode and the working reference electrode prior to specimen measurement.

In addition, some embodiments provide an electrode structure of the biosensor strip in which each of the strip recognition electrode and the specimen recognition electrode has a recognition reference electrode in common, to promote miniaturization of the biosensor strip.

In addition, some embodiments provide an electrode structure of the biosensor strip including a dummy electrode for bringing the specimen into contact with a specimen insertion passage in a width which is same to or larger than a pre-determined width, to improve dispensing spreadability defect for interface material properties.

However, technical problems to be solved by the present disclosure are not limited to the above problems and may be variously expanded without departing from the technical spirit and scope of the present disclosure.

### [TECHNICAL SOLUTION]

According to one embodiment, an electrode structure of a biosensor strip for specimen measurement may comprise a strip recognition electrode used to recognize whether the biosensor strip is inserted into a specimen measurement device, a specimen recognition electrode used to recognize the specimen, and a working electrode and a working reference electrode used to measure the specimen.

According to one aspect, the strip recognition electrode and the specimen recognition electrode may be characterized by separately being provided to operate electrically independent of the working electrode and the working reference electrode.

According to another aspect, a working boss which is formed in a region corresponding to a specimen insertion passage of the biosensor strip in the working electrode and at least two or more working reference bosses which are formed in a region corresponding to the specimen insertion passage in the working reference electrode may be characterized by being alternately arranged while protruding in one direction perpendicular to a direction in which the working electrode and the working reference electrode are formed to extend.

According to yet another aspect, a recognition reference electrode for each of the strip recognition electrode and the specimen recognition electrode, may be characterized by being commonly provided for the strip recognition electrode and the specimen recognition electrode.

According to yet another aspect, a specimen recognition boss which is formed in a region corresponding to the specimen insertion passage of the biosensor strip in the specimen recognition electrode and a recognition reference boss which is formed in a region corresponding to the specimen insertion passage of the biosensor strip in the recognition reference electrode, may be characterized by being arranged with facing to each other while protruding in one direction perpendicular to a direction in which the specimen recognition electrode and the recognition reference electrode are formed to extend.

According to yet another aspect, the electrode structure of the biosensor strip may be characterized by further comprising a dummy electrode provided on one side of a specimen insertion passage of the biosensor strip.

According to yet another aspect, the strip recognition electrode, the specimen recognition electrode, the working electrode, and the working reference electrode, may be characterized by being formed to extend in one direction while being spaced apart from each other in parallel.

According to one embodiment, a biosensor strip for specimen measurement may comprise a lower plate having an electrode structure including a strip recognition electrode used to recognize whether the biosensor strip is inserted into a specimen measurement device, a specimen recognition electrode used to recognize the specimen, and a working electrode and a working reference electrode used to measure the specimen, a middle plate disposed on an upper side of the lower plate and including a specimen insertion passage into which an enzyme compound that reacts with the specimen is inserted, and an upper plate disposed on an upper side of the middle plate and including an insertion hole for inserting the specimen into the specimen insertion passage.

According to one aspect, in the lower plate, the strip recognition electrode and the specimen recognition electrode may be characterized by separately being provided to operate electrically independent of the working electrode and the working reference electrode.

According to another aspect, in the lower plate, a recognition reference electrode for each of the strip recognition electrode and the specimen recognition electrode may be characterized by being commonly provided for the strip recognition electrode and the specimen recognition electrode.

According to yet another aspect, the lower plate may be characterized by further comprising a dummy electrode provided on one side of the specimen insertion passage of the biosensor strip.

According to one embodiment, a specimen measurement system may comprise a biosensor strip having an electrode structure including a strip recognition electrode used to recognize whether the biosensor strip is inserted into a specimen measurement device, a specimen recognition electrode used to recognize a specimen, and a working electrode and a working reference electrode used to measure the specimen, and the specimen measurement device that recognizes whether the biosensor strip is inserted, recognizes whether the specimen is in contact with the biosensor strip when the biosensor strip is inserted into the specimen measurement device, and applies a specimen measurement signal to the working electrode and the working reference electrode to measure a response signal for the specimen when the specimen is in contact with the biosensor strip.

According to one embodiment, a specimen measurement method performed by a specimen measurement system including a biosensor strip and a specimen measurement device may comprise recognizing whether the biosensor strip is inserted into the specimen measurement device using a strip recognition electrode included in the biosensor strip; recognizing whether a specimen is in contact with the biosensor strip using a specimen recognition electrode included in the biosensor strip, when the biosensor strip is inserted into the specimen measurement device; and measuring a response signal for the specimen by applying a specimen measurement signal to a working electrode and a working reference electrode included in the biosensor strip, when the specimen is in contact with the biosensor strip.

According to one aspect, as the strip recognition electrode and the specimen recognition electrode are separately provided to operate electrically independent of the working electrode and the working reference electrode, the measuring of the response signal for the specimen may be characterized by being performed independently of the recognizing of whether the biosensor strip is inserted into the specimen measurement device and the recognizing of whether the specimen is in contact with the biosensor strip.

According to another aspect, the specimen measurement method may be characterized by further comprising bringing the specimen into contact with a specimen insertion passage of the biosensor strip in a width which is same to or larger than a pre-determined width, using a dummy electrode provided on one side of the specimen insertion passage of the biosensor strip.

### [ADVANTAGEOUS EFFECTS OF THE INVENTION]

Some embodiments, by proposing an electrode structure of a biosensor strip, including a strip recognition electrode and a specimen recognition electrode that are separately provided to operate electrically independent of a working electrode and a working reference electrode, may prevent an electric double layer from being formed on surfaces of the working electrode and the working reference electrode prior to a specimen measurement to achieve a technical effect that improves specimen measurement accuracy.

Further, some embodiments, by proposing an electrode structure of a biosensor strip, in which each of a strip recognition electrode and a specimen recognition electrode has a recognition reference electrode in common, may achieve a technical effect that promotes miniaturization of the biosensor strip.

Further, some embodiments, by proposing an electrode structure of a biosensor strip including a dummy electrode for bringing a specimen into contact with a specimen insertion passage in a width which is same as or larger than a pre-determined width, may achieve a technical effect that improves dispensing spreadability defect for interface material properties.

However, effects of the present disclosure are not limited to the above effects and may be variously expanded in various ways without departing from the technical spirit and scope of the present disclosure.

### [DESCRIPTION OF THE DRAWINGS]

FIG. 1 is a drawing illustrating a specimen measurement system according to one embodiment.
FIG. 2 is a drawing illustrating a specimen collection device which is illustrated in FIG.1.
FIG. 3 is a drawing illustrating a biosensor strip which is illustrated in FIG. 1.
FIG. 4 is a drawing illustrating an electrode structure that a lower plate of the biosensor strip illustrated in FIG. 3 has.
FIG. 5 is a flowchart illustrating a specimen measurement method according to one embodiment.
FIG. 6 is a drawing illustrating an example for describing the specimen measurement method which is illustrated in FIG. 5.

### [BEST MODE]

Hereinafter, embodiments of the present disclosure will be described in detail with reference to the referred drawing. However, the present disclosure is not restricted or limited by the embodiments. Further, the same reference numerals depicted in each drawing represent the same components.

Further, terms used in the specification are used to properly express the preferred embodiments of the present disclosure, and the terms may vary depending on the intention of a viewer or an operator, or customs in the field to which the present disclosure belongs. Therefore, definitions of these terms should be made based on the content throughout this specification. For example, in the specification, a singular form also includes a plural form unless the context specifically states otherwise. Further, the term "comprise" and/or "comprising" used herein does not exclude the presence or addition of one or more other components, steps, operations, and/or elements in or to components, steps, operations, and/or elements mentioned above. Further, even though the terms such as first, second, etc. are used in this specification to describe various regions, directions, shapes, etc., the regions, directions, shapes should not be limited by these terms. These terms are merely used to distinguish one region, direction or shape from another region, direction or shape. Thus, a part referred to as a "first part" in one embodiment may be referred to as a "second part" in another embodiment.

Further, it should be understood that various embodiments of the present disclosure are different from each other but are not necessarily mutually exclusive. For example, specific shapes, structures, and characteristics described herein may be implemented in another embodiment without departing from the technical spirit and scope of the present disclosure in relation to one embodiment. Further, it should be understood that positions, arrangements, or configurations of individual components in the range of each presented embodiment may be changed without departing from the technical spirit and scope of the present disclosure.

Hereinafter, a biosensor strip, an electrode structure thereof, a specimen measurement system including the same, and a specimen measurement method using the same according to embodiments will be described with reference to the drawings.

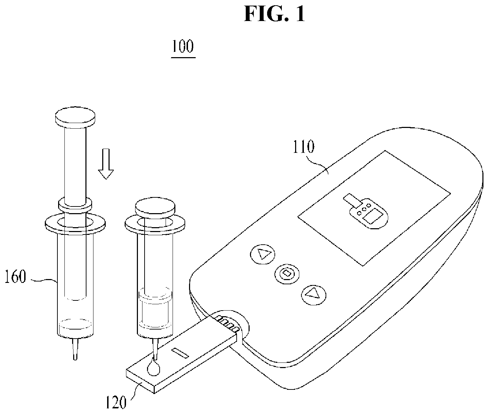

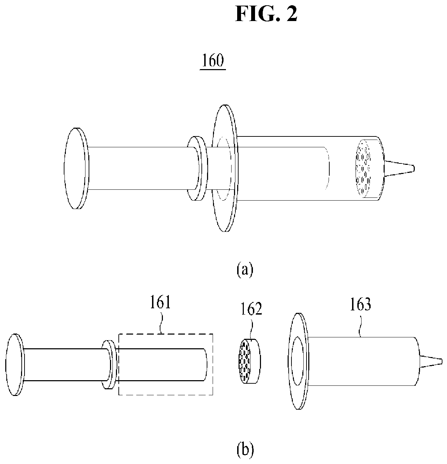

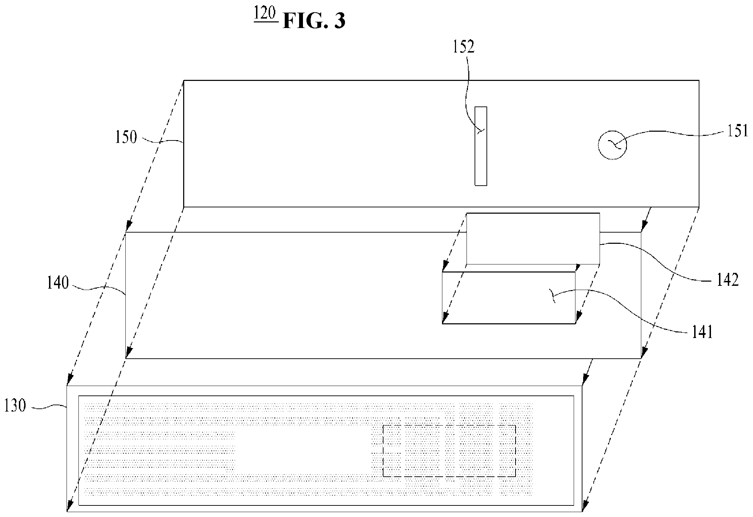

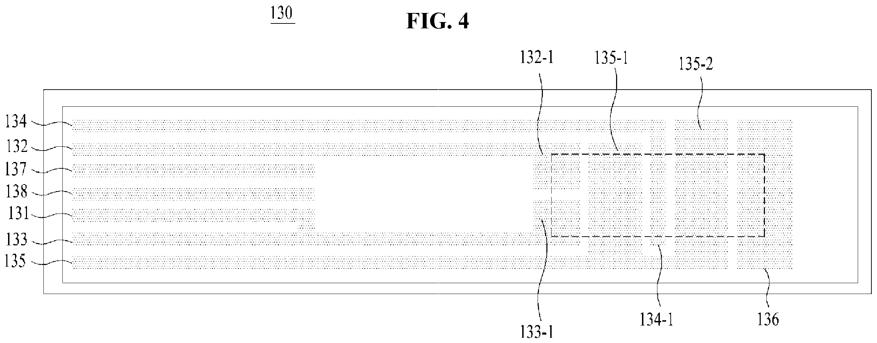

FIG. 1 is a drawing illustrating a specimen measurement system according to one embodiment, FIG. 2 is a drawing illustrating a specimen collection device which is illustrated in FIG. 1, FIG. 3 is a drawing illustrating a biosensor strip which is illustrated in FIG. 1, and FIG. 4 is a drawing illustrating an electrode structure that a lower plate of the biosensor strip which is illustrated in FIG. 3 has.

A specimen measurement system 100 according to one embodiment may include a specimen measurement device 110, a biosensor strip 120, and a specimen collection device 160.

As illustrated in FIG. 2, the specimen collection device 160 includes a specimen collection unit 161 including a specimen collection swab 161-1 for collecting a specimen, a filter 162, and a compression tube 163.

In this case, when the specimen is saliva, the specimen collection unit 161 may collect the saliva through the specimen collection swab 161-1, and after interfering substances included in the saliva are removed through the filter 162 inserted into the compression tube 163, the saliva collected by the specimen collection unit 161 may be provided to the outside, that is, the biosensor strip 120, through the compression tube 163.

That is, after the saliva is collected, the specimen collection device 160 may compress the specimen collection unit 161 in a direction of the compression tube 163 inside the compression tube 163 to remove the interfering substances included in the saliva through the filter 162.

The filter 162 may be composed of at least one layer. In particular, in the filter 162, the thickness of the layer to be produced or the number of layers may be adjusted in consideration of a degree of removal of the interfering substances to be removed from the specimen, and a degree of filtering may be adjusted in consideration of the size of the interfering substances to be removed from the specimen. As an example, when it is intended to remove large-sized molecules and foreign substances present in the saliva as interfering substances, the filter 162 may be formed to have a degree of filtering enough to remove the large-sized molecules and the foreign substances.

When the specimen collected by the specimen collection device 160 is in contact with and inserted through the specimen collection device 160, the biosensor strip 120 may perform a function of sensing information (e.g., glucose) to be measured from the specimen from which the interfering substances are removed. While being inserted into the specimen measurement device 110, the biosensor strip 120 may provide, to the specimen measurement device 110, a response signal in response to a strip recognition signal received from the specimen measurement device 110, a response signal in response to a specimen recognition signal received from the specimen measurement device 110, and a response signal in response to a specimen measurement signal received from the specimen measurement device 110. Accordingly, the specimen measurement device 110 may recognize insertion of the biosensor strip 120 and contact with the specimen and measure the specimen based on the response signals received from the biosensor strip 120.

As illustrated in FIG. 3, the biosensor strip 120 may be composed of a lower plate 130, a middle plate 140, and an upper plate 150. More specifically, the biosensor strip 120 may include the lower plate 130 in which an electrode structure is formed as illustrated in FIG. 4, the middle plate 140 disposed on an upper side of the lower plate 130 and including a specimen insertion passage 141 into which an enzyme compound that reacts with the specimen is inserted, and the upper plate 150 disposed on an upper side of the middle plate 140 and including an insertion hole 151 for inserting the specimen into the specimen insertion passage 141 and a discharge hole 152 for discharging air.

Here, since the specimen insertion passage 141 is obvious to those skilled in the art, a detailed description thereof will be omitted.

An enzyme compound 142 may include an enzyme for selectively reacting with glucose included in the specimen, a polymer for attaching the enzyme onto electrodes arranged on the lower plate 130, and a catalyst for promoting a reaction between the enzyme and the glucose.

In this case, the catalyst may include 1% to 10% of at least any one of ferrocene, a ferrocene derivative, quinone, a quinone derivative, hexaamine ruthenium (III) chloride, prussian blue, and ferricyanide (when prussian blue is used, the catalyst may include 0.001% to 5% of prussian blue), and the enzyme may include 0.01 wt% to 1.0 wt% of at least any one of glucose oxidase and glucose dehydrogenase (GDH), and the polymer may include 1 unit to 30 units of at least any one of chitosan, PVP, nafion, polyethylene glycol, poly vinyl pyrrolidone, poly vinyl alcohol, agarose, and trehalose.

An electrode structure in which a strip recognition electrode 131, a specimen recognition electrode 132, a recognition reference electrode 133, a working electrode 134, a working reference electrode 135, a dummy electrode 136, and spare electrodes 137 and 138 are included may be formed in the lower plate 130.

The strip recognition electrode 131 may be used to recognize whether the biosensor strip 120 is inserted into the specimen measurement device 110. As an example, the strip recognition signal is applied from the specimen measurement device 110 through the strip recognition electrode 131 and the recognition reference electrode 133, and the response signal in response thereto may be provided from the biosensor strip 120 to the specimen measurement device 110 through the strip recognition electrode 131 and the recognition reference electrode 133. Thus, the specimen measurement device 110 may recognize whether the biosensor strip 120 is inserted into the specimen measurement device 110 using the strip recognition electrode 131. Whether the biosensor strip 120 is inserted into the specimen measurement device 110 may be determined based on an intensity of the response signal in response to the strip recognition signal or whether the response signal is generated and provided.

The specimen recognition electrode 132 may be used to recognize the specimen. More specifically, the specimen recognition electrode 132 may be used to recognize whether the specimen is in contact with the biosensor strip 120. As an example, the specimen recognition signal is applied from the specimen measurement device 110 through the specimen recognition electrode 132 and the recognition reference electrode 133, and the response signal in response thereto may be provided from the biosensor strip 120 to the specimen measurement device 110 through the specimen recognition electrode 132 and the recognition reference electrode 133. Thus, the specimen measurement device 110 may recognize whether the specimen is in contact with the biosensor strip 120 using the specimen recognition electrode. Whether the specimen is in contact with the biosensor strip 120 may be determined based on an intensity of the response signal in response to the specimen recognition signal and whether the response signal is generated and provided.

A specimen recognition boss 132-1 formed in a region corresponding to the specimen insertion passage 141 of the biosensor strip 120 in the specimen recognition electrode 132 and a recognition reference boss 133-1 formed in a region corresponding to the specimen insertion passage 141 of the biosensor strip 120 in the recognition reference electrode 133 may be arranged to face each other while protruding in one direction perpendicular to a direction in which the specimen recognition electrode 132 and the recognition reference electrode 133 are formed to extend and being spaced apart from each other.

In this case, the recognition reference electrode 133 for each of the strip recognition electrode 131 and the specimen recognition electrode 132 may be provided in common for the strip recognition electrode 131 and the specimen recognition electrode 132. That is, the strip recognition electrode 131 and the specimen recognition electrode 132 may share the one recognition reference electrode 133. Accordingly, unlike a case in which the strip recognition electrode 131 and the specimen recognition electrode 132 each have the recognition reference electrode, miniaturization of the biosensor strip 120 may be possible.

The working electrode 134 and the working reference electrode 135 may be used to measure the specimen. Specifically, the specimen measurement signal may be applied from the specimen measurement device 110 through the working electrode 134 and the working reference electrode 135, and the response signal for the specimen in response thereto may be provided from the biosensor strip 120 to the specimen measurement device 110 through the working electrode 134 and the working reference electrode 135. Thus, the specimen measurement device 110 may measure the specimen by measuring the response signal for the specimen using the working electrode 134 and the working reference electrode 135. A specimen measurement result may be determined based on an intensity of the response signal in response to the specimen measurement signal or whether the response signal is generated and provided. For example, the specimen measurement result may represent several levels of measurement values depending on the intensity of the response signal in response to the specimen measurement signal.

A working boss 134-1 formed in a region corresponding to the specimen insertion passage 141 of the biosensor strip 120 in the working electrode 134 and two or more working reference bosses 135-1 and 135-2 formed in a region corresponding to the specimen insertion passage 141 in the working reference electrode 135 may be alternately arranged while protruding in one direction perpendicular to a direction in which the working electrode 134 and the working reference electrode 135 are formed to extend.

In this way, the working boss 134-1 and the working reference bosses 135-1 and 135-2 may be alternately arranged while the one working boss 134-1 is disposed between the two or more working reference bosses 135-1 and 135-2, and a region of the one working boss 134-1 may be formed smaller than a region of each of the two or more working reference bosses 135-1 and 135-2, so that when the specimen is in contact with the biosensor strip 120, the specimen measurement signal and the response signal in response to the specimen measurement signal may be accurately measured. Accordingly, specimen measurement accuracy may be improved.

The described strip recognition electrode 131, the specimen recognition electrode 132, the recognition reference electrode 133, the working electrode 134, and the working reference electrode 135 may be formed to extend in one direction while being spaced apart from each other in parallel.

In the electrode structure described above, the strip recognition electrode 131 and the specimen recognition electrode 132 may be separately provided to operate electrically independent of the working electrode 134 and the working reference electrode 135. Thus, electric double layers may be prevented from being formed on surfaces of the working electrode 134 and the working reference electrode 135 prior to the specimen measurement, and thereby the specimen measurement accuracy may be improved.

The dummy electrode 136 is for bringing the specimen into contact with the specimen insertion passage 141 in a width which is same as or larger than a pre-determined width to improve dispensing spreadability defect for interface material properties, and may be provided on one side of a region corresponding to the specimen insertion passage 141 in the lower plate 130.

The spare electrodes 137 and 138 are electrodes provided for spare purposes in the electrode structure of the described biosensor strip 120, and may be used as any one of the strip recognition electrode 131, the specimen recognition electrode 132, the recognition reference electrode 133, the working electrode 134, and the working reference electrode 135 as needed.

Although not illustrated in a separate drawing, the lower plate 130 on which the described electrode structure is formed may be formed as a plurality of metal layers overlap with each other. As an example, the lower plate 130 may be implemented as a quadruple structure in which an insulating substrate layer (not illustrated), a first metal layer (not illustrated) formed of copper on the insulating substrate layer, a second metal layer (not illustrated) formed of nickel on the first metal layer, and a third metal layer (not illustrated) formed of gold on the second metal layer overlap with each other. **In** this case, the electrode structure described above may be formed in each of the first metal layer, the second metal layer, the third metal layer, and the fourth metal layer.

As the biosensor strip 120 through which the specimen is introduced while being in contact with the biosensor strip 120 is inserted, the specimen measurement device 110 may sequentially apply the strip recognition signal, the specimen recognition signal, and the specimen measurement signal to the biosensor strip 120, may receive the response signals for each of the strip recognition signal, the specimen recognition signal, and the specimen measurement signal, and thereby may recognize that the biosensor strip 120 is inserted and the specimen is in contact with the biosensor strip 120 and measure the specimen.

A result of measuring the specimen may be provided as several levels of measurement values depending on the intensity of the response signal in response to the specimen measurement signal, and may be displayed through a display mean of the specimen measurement device 110 while having different graphic effects applied depending on the measurement values.

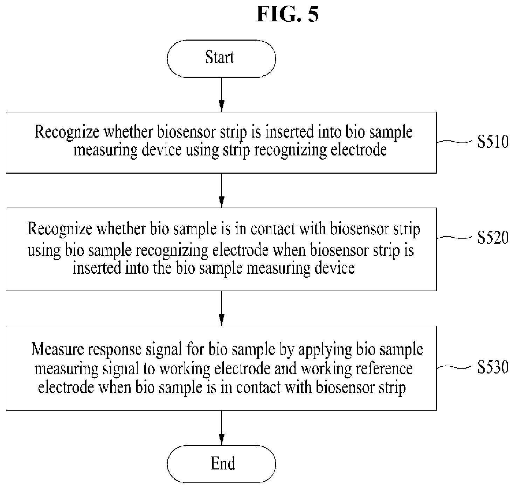

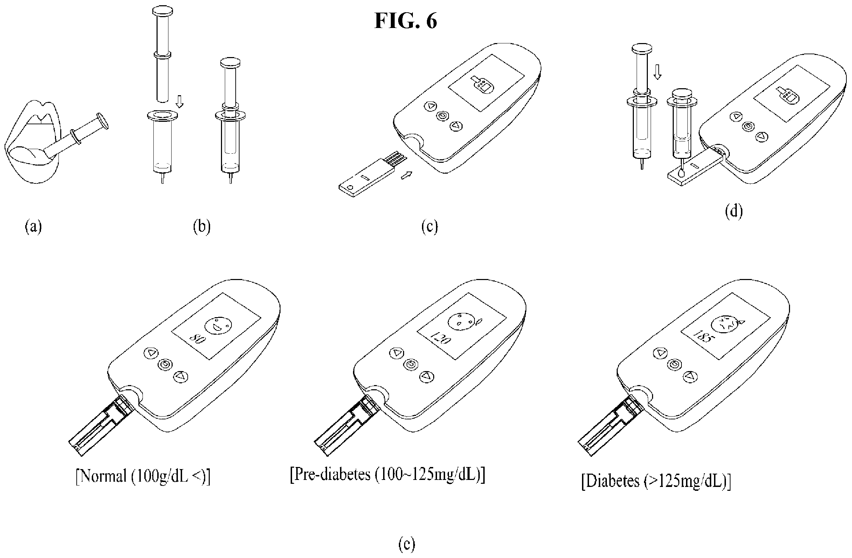

FIG. 5 is a flowchart illustrating a specimen measurement method according to one embodiment, and FIG. 6 is a drawing illustrating an example for describing the specimen measurement method which is illustrated in FIG. 5. It is assumed that the specimen measurement method, which will be described below, is performed mainly by the specimen measurement system 100 described above with reference to FIGS. 1 to 4.

Before operations S510 to S530, the specimen measurement system 100 (more precisely, the specimen collection device 160) may collect saliva of a subject using the specimen collection unit 161 of the specimen collection device 160, may insert the collected saliva into the compression tube 163 provided with the filter 162, and thereby may remove interfering substances from the specimen which is the saliva as illustrated in (a), (b) of FIG. 6. Then, the biosensor strip 120 may be inserted into the specimen measurement device 110 as illustrated in (c) of FIG. 6, the specimen may be inserted and introduced into the insertion hole 151 of the biosensor strip 120 from the specimen collection device 160 as illustrated in (d) of FIG. 6, and thereby the specimen may be in contact with the specimen insertion passage 141 of the biosensor strip 120.

In this case, the specimen which is inserted and introduced into the biosensor strip 120 may be in contact with the specimen insertion passage 141 of the biosensor strip 120 in a width which is same as or larger than a pre-determined width by the dummy electrode 136 provided on one side of the specimen insertion passage 141 of the biosensor strip 120.

In operation S510, the specimen measurement system 100 (more precisely, the specimen measurement device 110) may recognize whether the biosensor strip 120 is inserted into the specimen measurement device 110 using the strip recognition electrode 131 included in the biosensor strip 120. More specifically, the specimen measurement device 110 may apply the strip recognition signal through the strip recognition electrode 131 and the recognition reference electrode 133, may be provided with the response signal in response thereto from the biosensor strip 120 through the strip recognition electrode 131 and the recognition reference electrode 133, and thereby may recognize and determine whether the biosensor strip 120 is inserted into the specimen measurement device 110.

In operation S520, when the biosensor strip 120 is inserted into the specimen measurement device 110 (when it is recognized in operation S510 that the biosensor strip 120 is inserted into the specimen measurement device 110), the specimen measurement system 100 (more precisely, the specimen measurement device 110) may recognize whether the specimen is in contact with the biosensor strip 120 using the specimen recognition electrode 132 included in the biosensor strip 120. Specifically, the specimen measurement device 110 may apply the specimen recognition signal through the specimen recognition electrode 132 and the recognition reference electrode 133, may be provided with the response signal in response thereto from the biosensor strip 120 through the specimen recognition electrode 132 and the recognition reference electrode 133, and thereby may recognize and determine whether the specimen is in contact with the biosensor strip 120.

In operation S530, when the specimen is in contact with the biosensor strip 120 (when it is recognized in operation S520 that the biosensor strip 120 is in contact with the biosensor strip 120), the specimen measurement system 100 (more precisely, the specimen measurement device 110) may apply the specimen measurement signal to the working electrode 134 and the working reference electrode 135 included in the biosensor strip 120 to measure the response signal for the specimen as illustrated in (e) of FIG. 6. More specifically, the specimen measurement device 110 may apply the specimen measurement signal through the working electrode 134 and the working reference electrode 135, may be provided with the response signal in response thereto from the biosensor strip 120 through the working electrode 134 and the working reference electrode 135, and thereby may measure the response signal in response to the specimen measurement signal to measure the specimen.

In particular, as the strip recognition electrode 131 and the specimen recognition electrode 132 are separately provided to operate electrically independent of the working electrode 134 and the working reference electrode 135, the operation S530 of measuring the response signal for the specimen may be performed independently of the operation S510 of recognizing whether the biosensor strip 120 is inserted into the specimen measurement device 110 and the operation S520 of recognizing whether the specimen is in contact with the biosensor strip 120.

As described above, although the embodiments have been described with reference to the limited embodiments and the limited drawings, it is appreciated that various modifications and changes may be made based on the above description by those skilled in the art. For example, even though the described technologies are performed in an order different from the described method, and/or the components such as the described system, structure, device, and circuit, etc. are coupled or combined in a form different from the described method, or are replaced or substituted by other components or equivalents, appropriate results may be achieved.

Therefore, other implementations, other embodiments, and those equivalent to the appended claims also belong to the scope of the appended claims.

## Claims

1. An electrode structure of a biosensor strip for specimen measurement, the electrode structure comprising:
a strip recognition electrode used to recognize whether the biosensor strip is inserted into a specimen measurement device;
a specimen recognition electrode used to recognize the specimen; and
a working electrode and a working reference electrode used to measure the specimen.

2. The electrode structure of the biosensor according to claim 1, wherein the strip recognition electrode and the specimen recognition electrode are **characterized by** separately being provided to operate electrically independent of the working electrode and the working reference electrode.

3. The electrode structure of the biosensor according to claim 1, wherein a working boss which is formed in a region corresponding to a specimen insertion passage of the biosensor strip in the working electrode and at least two or more working reference bosses which are formed in a region corresponding to the specimen insertion passage are **characterized by** being alternately arranged while protruding in one direction perpendicular to a direction in which the working electrode and the working reference electrode are formed to extend.

4. The electrode structure of the biosensor according to claim 1, wherein a recognition reference electrode for each of the strip recognition electrode and the specimen recognition electrode is **characterized by** being commonly provided for the strip recognition electrode and the specimen recognition electrode.

5. The electrode structure of the biosensor according to claim 4, wherein a specimen recognition boss which is formed in a region corresponding to the specimen insertion passage of the biosensor strip in the specimen recognition electrode and a recognition reference boss which is formed in a region corresponding to the specimen insertion passage of the biosensor strip in the recognition reference electrode, are **characterized by** being arranged with facing each other while protruding in one direction perpendicular to a direction in which the specimen recognition electrode and the recognition reference electrode are formed to extend.

6. The electrode structure of the biosensor according to claim 1, being **characterized by** further comprising:
a dummy electrode provided on one side of a specimen insertion passage of the biosensor strip.

7. The electrode structure of the biosensor according to claim 1, wherein the strip recognition electrode, the specimen recognition electrode, the working electrode, and the working reference electrode, are **characterized by** being formed to extend in one direction while being spaced apart from each other in parallel.

8. A biosensor strip for specimen measurement, the biosensor strip comprising:
a lower plate having an electrode structure including a strip recognition electrode used to recognize whether the biosensor strip is inserted into a specimen measurement device, a specimen recognition electrode used to recognize a specimen, and a working electrode and a working reference electrode used to measure the specimen;
a middle plate disposed on an upper side of the lower plate and including a specimen insertion passage into which an enzyme compound that reacts with the specimen is inserted; and
an upper plate disposed on an upper side of the middle plate and including an insertion hole for inserting the specimen into the specimen insertion passage.

9. The biosensor strip according to claim 8, wherein, in the lower plate, the strip recognition electrode and the specimen recognition electrode, are **characterized by** being separately provided to operate electrically independent of the working electrode and the working reference electrode.

10. The biosensor strip according to claim 8, wherein, in the lower plate, a
recognition reference electrode for each of the strip recognition electrode and the specimen recognition electrode, is **characterized by** being commonly provided for the strip recognition electrode and the specimen recognition electrode.

11. The biosensor strip according to claim 8, being **characterized in that** the lower plate further comprises:
a dummy electrode provided on one side of the specimen insertion passage of the biosensor strip.

12. A specimen measurement system, the system comprising:
a biosensor strip having an electrode structure including a strip recognition electrode used to recognize whether the biosensor strip is inserted into a specimen measurement device, a specimen recognition electrode used to recognize a specimen, and a working electrode and a working reference electrode used to measure the specimen; and
the specimen measurement device which recognizes whether the biosensor strip is inserted, recognizes whether the specimen is in contact with the biosensor strip when the biosensor strip is inserted into the specimen measurement device, and applies a specimen measurement signal to the working electrode and the working reference electrode to measure a response signal for the specimen when the specimen is in contact with the biosensor strip.

13. A specimen measurement method performed by a specimen measurement system including a biosensor strip and a specimen measurement device, the specimen measurement method comprising:
recognizing whether the biosensor strip is inserted into the specimen measurement device using a strip recognition electrode included in the biosensor strip;
recognizing whether a specimen is in contact with the biosensor strip using a specimen recognition electrode included in the biosensor strip, when the biosensor strip is inserted into the specimen measurement device; and
measuring a response signal for the specimen by applying a specimen measurement signal to a working electrode and a working reference electrode included in the biosensor strip, when the specimen is in contact with the biosensor strip.

14. The specimen measurement method according to claim 13, wherein, as the strip recognition electrode and the specimen recognition electrode are separately provided to operate electrically independent of the working electrode and the working reference electrode, the measuring of the response signal for the specimen is **characterized by** being performed independently of the recognizing of whether the biosensor strip is inserted into the specimen measurement device and the recognizing of whether the specimen is in contact with the biosensor strip.

15. The specimen measurement method according to claim 13, being **characterized by** further comprising: bringing the specimen into contact with a specimen insertion passage of the biosensor strip in a width which is same to or larger than a pre-determined width, using a dummy electrode provided on one side of the specimen insertion passage of the biosensor strip.
